# EUROPEAN PATENT APPLICATION

(11) **EP 3 843 105 A1**
(43) Date of publication of application: **30.06.2021**
(21) Application number: 19219358.9
(22) Date of filing: 23.12.2019
(51) Int. Cl.: G16H 40/60, A61M 11/00

(54) **CONTROL DEVICE FOR AEROSOL NEBULIZER SYSTEM**

(71) Applicant: PARI Pharma GmbH, 82319 Starnberg (DE)
(72) Inventor: Fuchs, Carola, 82061 Neuried (DE); Finke, Matthias, 82152 Planegg (DE); Reimpell, Philippine, 82340 Feldafing (DE); Fiebig, David, 80337 München (DE)
(74) Representative: Hoffmann Eitle

(57) **Abstract**

Provided is a control device 20, 20a for controlling an operation of an aerosol nebulizer system 30, said aerosol nebulizer system 30 comprising an aerosol generator 31 for nebulizing a liquid or an aerosol source for dispensing aerosol, said control device 20, 20a comprising: a communication unit 21, 21a, configured to establish a first wireless communication connection and to perform first data transmission with the aerosol nebulizer system 30, a control unit 22, 22a, configured to evaluate a progression of identifications received via the first data transmission and received in association with aerosol nebulizer data units respectively related to a usage of the aerosol nebulizer system 30.

## Description

### TECHNICAL FIELD

The present invention relates to a control device for controlling an operation of an aerosol nebulizer system, as well as an aerosol nebulizer system and a server.

### BACKGROUND

A high adherence to therapy is important for effective treatment of, for example, cystic fibrosis (CF) and at the same time difficult to achieve for the patients due to the timely burden of the many necessary therapies. CF patients require, for example, up to 4 hours daily for therapy activities which often leads to stressful situations, in particular in families with CF kids.

Studies have shown that adherence typically declines over time, for example by 9 % on average between the first and third month of such studies. It was also shown in such studies that patients with less burdensome treatment regimens were more adherent than those with more daily prescribed nebulizations. In accordance with this finding, patients who were prescribed antibiotics for nebulization were less adherent than those who did not have to nebulize antibiotics as part of their treatment plan. Patients with high adherence rates (>75%) had a better forced expiratory volume in one second (FEV₁) development with an increase of 3.9 % compared to an average decline of FEV₁ of the total patient group of 1% over the monitored period of 6 months.

As such, it was concluded that nebulization therapy adherence is an important parameter associated with better health outcomes for the patient. Using new technologies and better awareness of adherence tracking going on in the background may motivate patients to be more adherent, engage with their disease management and follow their therapy more diligently.

Conventional aerosol nebulizers are known to have an aerosol generator to generate an aerosol from a liquid medication. To improve the usability of the aerosol nebulizers for the user/patient, an external control device may be used. Such a control device for controlling an operation of an aerosol nebulizer system may be a mobile device, for example a smartphone implementing an application software program (an APP or a mobile APP), and may comprise a processor, a memory, for example in the form of a non-volatile memory, a display, and an input unit which may be configured in the form of a keyboard, individual buttons, or a touch-sensitive surface in the display.

Such an external control device may additionally implement a suitable interface to establish a wireless communication connection with the aerosol nebulizer to transfer configuration data to the aerosol nebulizer to appropriately set and control the operation of the aerosol generator. Similarly, nebulization data and/or measurement data (also referred to as therapy-related data) may be generated by one or more sensor units of the aerosol nebulizer and may be transferred from the aerosol nebulizer to the external control device via the wireless communication connection. Such nebulization data and/or measurement data may, for example, be generated by an internal control unit of the aerosol nebulizer or the one or more sensor units that are mounted at the aerosol nebulizer and/or are connected with the aerosol nebulizer. An example of such an external control device is an external computing device, for example in the form of a smartphone or a PDA as described in DE 102 43 371 A1 or US 2006/0237001 A1.

Such an external control device may further exhibit a telecommunication module and may thus further offer the capability to transfer the configuration data and/or inhalation/nebulization/measurement data (e.g. sensor data) via the internet or a cloud from/to a (central) data base or a (central) data server (also referred to as server or backend server in the following), for example for telemedicine purposes and for the purpose of (centralized) electronic health records, or to an individual recipient other than the user itself, for example to the physician of the patient. As such, also the server may be regarded as a control device for controlling an operation of an aerosol nebulizer system.

### SUMMARY OF THE INVENTION

### TECHNICAL PROBLEM

In order to improve the medical success of the inhalation therapy by improving adherence to a therapy protocol, by dynamically adapting the therapy protocol, and by providing real-time therapy-related functionality, such as patient training and support at the control device during the inhalation process, it is important that all relevant data of the usage of the aerosol nebulizer system (usage data in the following), e.g. data related to inhalation treatments, sensor data, cleaning data, charging data, status data, user identification data etc. are completely available for analysis, for example at a central monitoring server (such as a server, a backend server, a web portal or the like).

However, the devices outside the aerosol nebulizer system (e.g. control device such as mobile device (smartphone or the like) or server) do not have prior knowledge as to when usage data are transmitted or about how many of such usage data should be received. A conventional control device therefore has no mechanism to determine whether all usage data have actually been received. Such usage data of the aerosol nebulizer system may be lost due to the nature of the wireless communication connection between the control device and the aerosol nebulizer system.

In addition or alternatively, if a plurality of control devices are used, for example because the APP has been installed on a plurality of control devices, such as a smartphone, a tablet, a wearable device, then a plurality of control devices may be used to provide the usage data to the central monitoring server. In such a situation, there may be a risk that an integrity of the usage data or, in other words, data completeness of the usage data is not guaranteed, for example because a first subset of the usage data is provided via a first control device while a second subset of usage data fails to be provided via the second control device which a user may use at a later point in time to access the data at the aerosol nebulizer system.

Against this background, it is an objective of the present invention to provide a control device, an aerosol nebulizer system and a server that overcome the above described technical disadvantages.

### SOLUTION

The features of a control device according to the present invention are defined in claim 1. Advantageous embodiments are described in the corresponding dependent claims.

Further, the features of an aerosol nebulizer system according to the present invention are defined in claim 13. Advantageous embodiments are described in the corresponding dependent claims.

Further, the features of a server according to the present invention are defined in claim 16. Advantageous embodiments are described in the corresponding dependent claims.

Further, the features of a computer program (e.g. a mobile application or APP) according to the present invention are defined in claim 22, while the features of a non-transitory computer-readable storage medium and a signal carrying the computer program are defined in claims 23 and 24, respectively.

### BRIEF DESCRIPTION OF THE DRAWINGS

**Fig. 1** shows a schematic illustration of a control device (e.g. a mobile device or a server) in communication interaction with an aerosol nebulizer system according to an embodiment.
**Fig. 2** shows a schematic illustration of a control device (e.g. a mobile device or a server) in communication interaction with an aerosol nebulizer system and a third device according to another embodiment.
**Fig. 3** shows a circular buffer according to an example of a storage unit at the aerosol nebulizer system according to an embodiment.
**Fig. 4** shows a data structure for implementing an association between aerosol nebulizer data units and corresponding identifications according to an embodiment.
**Fig. 5** shows a conceptional overview of the data integrity mechanism according to an embodiment.

### DESCRIPTION OF THE EMBODIMENTS

Embodiments of the present invention are described with reference to the Figures. It is noted that the following description should not be construed as limiting the invention. In the following, similar or same reference signs indicate similar or same elements or operations.

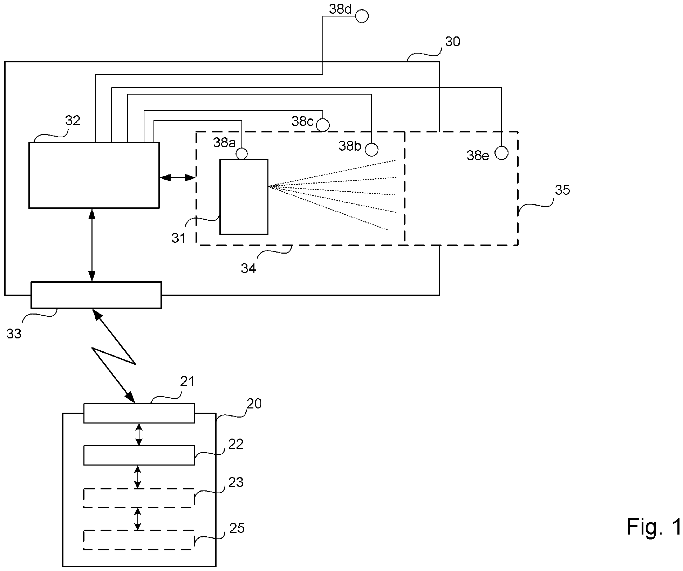

**Fig. 1** shows a control device 20 according to an embodiment in combination and communication interaction with an aerosol nebulizer system 30.

Here, the control device 20 may be a mobile device or a server.

Further, the aerosol nebulizer system 30 may comprise an aerosol generator 31. Here, the aerosol generator 31 may be a nebulizer, an atomizer, a humidifier, a pneumatic nebulizer, an electronic nebulizer, an ultrasonic nebulizer, an electro-hydrodynamic nebulizer, an electrostatic nebulizer, a jet nebulizer, a humidifier-nebulizer for ventilation devices, a metered-dose inhaler (MDI), a dry-powder inhaler (DPI), a spacer with MDI, a valved holding chamber with MDI or the like. Preferably the aerosol generator is a membrane nebulizer, more preferably a vibrating membrane nebulizer, e.g. an electronic vibrating membrane nebulizer.

In particular, the aerosol generator may be an electronic nebulizer, e.g. a piezo-electrically driven nebulizer, i.e. a nebulizer driven by a piezo-electric element.

The aerosol generator may be a jet nebulizer employing pressurized air and/or a compressor, such as those disclosed in US 5,957,389 and US -A-2007/0068513, the contents of which are hereby incorporated herein by reference in their entirety.

The aerosol generator may be a vibrating membrane nebulizer, such as those disclosed in EP-A-2 030 644, US-A-2012/0085344 and US-A-2013/0112197, the contents of which are hereby incorporated herein by reference in their entirety.

The aerosol generator may be a continuous aerosol generator, e.g. a continuously operated nebulizer. In particular, the aerosol generator may be a vibrating membrane nebulizer, e.g. a vibrating membrane nebulizer with a mixing chamber or aerosol chamber. The mixing chamber may have an inhalation valve that allows ambient air to flow into the mixing chamber during an inhalation phase, while preventing aerosol from escaping during an exhalation phase. Further, the mixing chamber may have an exhalation valve that allows discharge of the patient's respiratory air during the exhalation phase, while preventing an inflow of ambient air during the inhalation phase.

The aerosol generator may allow for aerosol losses occurring during exhalation of a regularly breathing user or patient to be minimized like described in US-A-2006/0054166. The aerosol generator may produce an aerosol continuously.

The exhalation valve may ensure that the patient's exhaled air is vented to the surroundings without significantly reaching the mixing chamber. During the exhalation phase, the continuously operating aerosol generator may accumulate or concentrate the aerosol in the mixing chamber, so that during an inhalation phase not only the amount of aerosol generated due to the continuous production is available for the inhalation, but at the beginning of the inhalation phase an aerosol bolus can be inhaled, which is available because of aerosol accumulation during the exhalation phase. Nebulizers adopting such an approach are also disclosed in EP-A-1 927 373 and US-A-2012/0037154

The aerosol generator, as described, is suitable to nebulize a liquid, which is held in a reservoir (not shown) of the aerosol nebulizer system 30. The liquid may be a fluid that contains at least one active pharmaceutical ingredient, medical drug and/or at least one therapy relevant fluid (such as a sodium chloride solution), and/or a fluid from which an aerosol is generated for the purpose of assisted diagnosis (such as a fluorescent aerosol, a mono-disperse aerosol, or the like). The liquid/fluid is transformed into an aerosol by the aerosol generator 31, which subsequently enters e.g. a mixing chamber 34 that is connected to a mouthpiece 35. From the mouthpiece, the patient/user inhales the aerosol into the respiratory tract, like lung, throat, nose or sinuses, to perform a medical treatment according to a prescribed aerosol therapy protocol. Further, the aerosol generator 31 (Fig. 1) operates according to configuration data that may be provided from the control device 20 via a communication interface 33 or may be pre-set at a processing unit 32 of the aerosol nebulizer system 30. Conversely, the aerosol nebulizer system 30 transmits (first data transmission) usage data via the established first wireless communication connection to the control device 20. As explained above, the usage data of the aerosol nebulizer system may include one or more data sets and, in particular, one or more data sets of sensor data (generated by one or more sensor units 38a, 38b, 38c, 38d, 38e at or near the aerosol nebulizer system 30), status data (e.g. indicating an operation status of one or more components of the aerosol nebulizer system 30, indicating a charging state or charging operation of a battery of the aerosol nebulizer system 30), and/or user data (e.g. indicating a user identification).

Here, the first wireless communication connection may be one of a Bluetooth connection, a Bluetooth Low Energy connection, a near field connection, a WiFi or WLAN connection, an infrared connection, a LoRa connection (a low power wide area network connection intended for wireless battery-operated Internet of things devices), a radio connection such as LTE, 5G, or the like. The data rate of Bluetooth Low Energy, for example, may be lower than 200 kBit/s, or even lower than 100 kBit/s. In addition, a wireless LoRa connection may have a data rate as low as 0.3 - 50 kBit/s.

Here, using the above radio connection, the aerosol nebulizer system 30 may also directly communicate with a server as the control device 20 to transmit the configuration data and/or usage data. In such a scenario, the server may operate as the control device 20.

Further, the configuration data and/or the usage data may be encrypted to avoid unauthorized access. In addition, the configuration data and/or the usage data of the aerosol nebulizer system may be transmitted in one or more single data units. For example, the usage data of the aerosol nebulizer system (stored in the aerosol nebulizer system 30, as further discussed below) may be transmitted using one or more data packets (each such data packet representing a single data unit). With regard to each individual single data unit, a first data integrity mechanism may be applied by implementing an error correction functionality. It is noted that this first data integrity mechanism is a mechanism to ensure that data of a single data unit (e.g. a single data packet) are correctly transmitted, for example by adding checksums, to the single data unit.

For establishing the first wireless communication connection, the control device 20 (mobile device or server) is provided with a communication unit 21 and the aerosol nebulizer system 30 is provided with the communication interface 33, as shown in Fig. 1. It is emphasized here that the first wireless communication connection to the control device 20 typically is not established continuously and may not be established, for example, when the control device 20 is a mobile device (or the APP on the control device 20) and is not used or not started or switched on by the user/patient yet. Likewise, if the control device 20 is a server, then the first wireless communication connection may be switched off. But even if there is no established first wireless communication interaction, the user may use the aerosol nebulizer system 30 according to the prescribed therapy protocol or the aerosol nebulizer system 30 may operate, for example recharging the battery, cleaning the aerosol generator, or the like. In this case, the usage data (as explained above) are stored in the aerosol nebulizer system 30 (as will be explained below) for a subsequent transmission to the control device 20 when the first wireless communication connection is established again.

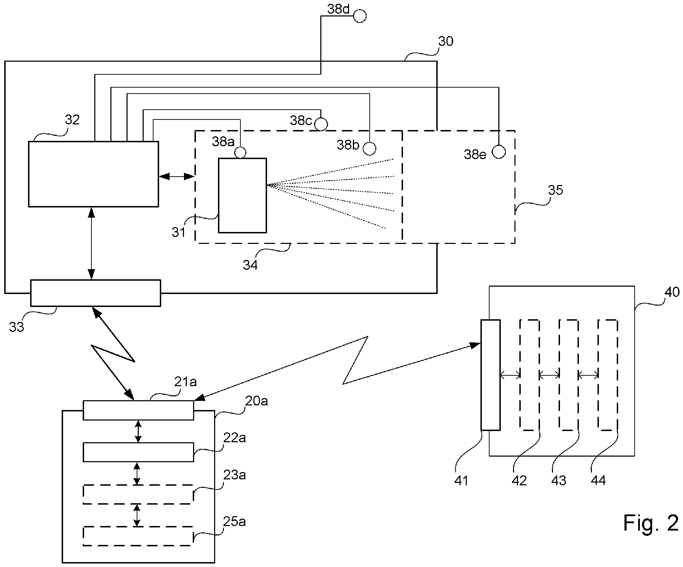

**Fig. 2** shows a control device 20a according to another embodiment in combination and communication interaction with the aerosol nebulizer system 30 and with an external third device 40. In such an embodiment, the control device 20a may be a mobile device or a server (as explained above with regard to **Fig. 1**) and performs data exchange with the external third device 40 (as will be explained below).

Here, according to a first alternative embodiment, if the control device 20a is a mobile device, then the external third device 40 may be a server. According to a second alternative embodiment, if the control device 20a is a server, then the external third device 40 may be a mobile device.

As shown in **Fig. 2**, the communication unit 21a of the control device 20a (mobile device or server), in addition to establishing the first wireless communication connection, as described above with regard to communication unit 21 in **Fig. 1**, may also be configured to establish a second wireless communication connection and to perform second data transmission with the external third device (server or mobile device) 40. The second wireless communication connection may be different from the first wireless communication connection, and may, for example, as in the above first alternative embodiment, involve a radio connection to a network (internet, cloud, or the like) to which a server 40 is connected. As will be further explained below, in this case, the server 40 may be one or more servers, one or more backend servers, one or more cloud servers, a web portal or another central unit within an IT network.

In general, the second wireless communication connection is established for performing second data transmission which involves transmitting the usage data of the aerosol nebulizer system 30 to the external third device (server or mobile device) 40 and may also include transmitting the configuration data from the external third device 40.

In the embodiments shown in **FIGs. 1** and **2**, the aerosol nebulizer system 30 comprises five sensor units 38a, 38b, 38c, 38d, 38e but the aerosol nebulizer system 30 is not limited to these five sensor units and may comprise more or less sensor units. In general, the sensor units supply measuring signals of parameters relevant for the therapy to the processing unit 32 which generates examples of the usage data of the aerosol nebulizer system from said measuring signals by means of a signal-based processing (e.g. analog-digital conversion, filtering, amplification) of the measuring signals. The usage data is released to the communication interface 33 by the processing unit 32 so as to be wirelessly transmitted as one or more aerosol nebulizer data units to the control device 20, 20a via the established first wireless communication connection.

The sensor units 38a, 38b, 38c, 38d, and 38e shown in **FIGs. 1** and **2** represent examples of different sensor units which generate measuring signals. For example, sensor unit 38a may detect the operation state of the membrane aerosol generator 31, for example the power consumption, temperature and/or current output and/or consumption of aerosol, the filling status of the liquid reservoir or another therapy-relevant parameter of the aerosol generator 31. The operation state of the membrane aerosol generator and/or the therapy-relevant parameter may be measured or calculated directly or indirectly from the sensor signals. The sensor unit 38b may detect the presence of aerosol and the aerosol density or temperature in the mixing chamber 34. The sensor unit 38c may be an environmental condition sensor unit to detect, for example, the temperature, humidity, pollution of ambient air and/or the pressure of the ambient air or a sensor to identify the tilting of the nebulizer or a sensor to identify the user/patient. Further, sensor unit 38d may detect, for example, the respiratory flow of the patient. Such a breathing maneuver sensor unit may determine a breathing (exhaling and inhaling) maneuver and a respiratory flow of the user/patient during inhalation of the generated aerosol and (optionally) during exhalation. In addition, sensor unit 38e may detect an actual contact of the user/patient with the mouthpiece 35. Naturally, the mouthpiece 35 could be any kind of an interface, like for example a mouthpiece, a face mask, nasal prongs, a tube whereas each contact area detects an actual contact of the user/patient like for example a contact surface, a display, a button, a handle and the like, as well as each contact area to detect an actual contact of the user/patient like for example a contact surface, a display, a button, a handle and the like.

The aerosol nebulizer system is not limited to the examples of the sensor units described above. For example, the aerosol nebulizer system may be provided with a sensor unit to determine a charging state of a battery of the aerosol nebulizer system. As explained above, the charging state may be another example of the usage data of the aerosol nebulizer system. Such a sensor unit may additionally determine when the charging state is or is expected to be above or below a certain threshold so that the usage data may also include an indication to inform the user that the aerosol nebulizer system is sufficiently charged or needs to be recharged.

As indicated above, the aerosol nebulizer system 30 according to the invention can be equipped with more or less sensor units for generating more or less usage data (one or more aerosol nebulizer data units). For example, an aerosol sensor unit may further determine one or more parameters of the generated aerosol such as aerosol density, a derivative of aerosol density over time to e. g. estimate an output rate of the aerosol generator, i. e. the amount of liquid aerosol released from the aerosol generator per unit of time, or aerosol quality, for example a statistical property of the aerosol droplets correlating e. g. with a mass median diameter (MMD), a mass median aerodynamic diameter (MMAD), a standard deviation from the MMD or MMAD, GSD (geometric standard deviation) or the like.

Further, a user recognition sensor unit may be provided to identify the current user of the aerosol nebulizer. As will be further discussed below, user recognition may be based on a code, icon, a smartcard, facial recognition, fingerprint, iris scan, breath analysis and/or special features of the sensor units. In addition, the user recognition may also be achieved via receiving identification data from the present control device, receiving identification data based on an app, or receiving identification data from an external device, such as a medical device (hearing aid device, cardiac pacemaker, an insulin pump) or vehicle which itself is provided with a capability to scan and recognize a user. Here, the user recognition data is another example of the usage data (one or more aerosol nebulizer data units) of the aerosol nebulizer system.

In addition, an aerosol generator identification sensor unit may identify a specific type of aerosol generator currently used at the aerosol nebulizer system, and a medication identification sensor unit to identify the type of medication that is currently used when generating aerosol. This identification may be based on an optical spectral measurement (within the liquid or within the aerosol droplets) or may be based on a RFID chip or another storage element being provided on an ampule containing the liquid, an electrical resistance measurement within the fluid, or the like. Here, the aerosol generator identification data is another example of the usage data (one or more aerosol nebulizer data units) of the aerosol nebulizer system.

In addition, an aerosol nebulizer battery sensor unit may be provided in order to determine a current state of charge (SOC) of a battery or a rechargeable battery, previous charging and/or discharging cycles, a battery voltage of the battery, respective battery voltages of battery cells, and the like. This may be achieved by measuring a cell voltage, a charging and/or discharging current, a charging and/or discharging time, and a comparison with respective target values which may be provided in a lookup table. In addition, when inserting a new battery cell, the new electric parameters (voltage, current, SOC) may be compared with such target values. Such data as to the SOC or other data related to an operation state of an element of the aerosol nebulizer system is another example of the usage data (one or more aerosol nebulizer data units) of the aerosol nebulizer system.

In addition, the aerosol nebulizer system may also be provided with a sensor to detect a geographic position of the aerosol nebulizer system, such as a GPSdetector, as another example of such usage data (one or more aerosol nebulizer data units). Such a detector is useful for setting an appropriate time zone and to adapt the therapy according to different geographic positions.

The control device 20, 20a according to **Figs. 1** and **2** may be a mobile device such as a smartphone, a tablet, a personal digital assistant (PDA), a netbook, a notebook, a laptop, a personal computer, a wearable device such as a smart watch, smart glasses, a running computer, hearing aid device, a voice controlled device or an implanted medical device, like an insulin pump computer or another portable device. Alternatively, a server may operate as the control device 20, 20a, as described above.

The control device 20, 20a of the aerosol nebulizer system 30 according to an embodiment may be equipped with an application program (e.g. an APP) for controlling an operation of the aerosol nebulizer system 30, for example, if the control device 20, 20a is a mobile device. The APP may control the operation of the aerosol nebulizer system 30 by transferring configuration data to the aerosol nebulizer system 30. The configuration data may determine a specific operation mode or condition of the aerosol nebulizer system 30, for example with regard to the electrical configuration parameters (voltage, current, duty cycle or ratio, duty ratio and/or frequency) of the aerosol generator or may provide configuration parameters with regard to the setting of any one of the sensors. The configuration data therefore may or may not influence the aerosol generation performance.

Alternatively, if the control device 20, 20a is a server, then an application program may be installed at the server and may control the operation of the aerosol nebulizer system 30 by transferring configuration data to the aerosol nebulizer system 30.

The application program may be stored in a memory unit 25, 25a of the control device 20, 20a and may be started from there within the framework of the operating system of the control device 20, 20a. Furthermore, it is alternatively possible to transfer an application program into the memory unit 25, 25a of the control device 20, 20a via an interface, for example the communication unit 21, 21a or an additional interface (RS-232, USB, FireWire, or the like) and to start it from there within the framework of the operating system of the control device 20, 20a. Finally, such an application program may be loaded into the memory unit 25, 25a of the control device 20, 20a via a remote data connection unit (not shown), for example downloaded via the Internet, and may be started from there within the framework of the operating system of the control device 20, 20a.

When switching on the aerosol nebulizer system 30, one or more of the sensor units 38a, 38b, 38c, 38d and 38e may generate sensor signals for the processing unit 32, which generates the usage data (one or more aerosol nebulizer data units) from the measuring signals by means of a signal-based processing of said measuring signals.

The aerosol nebulizer system 30 is provided with an aerosol generator 31 for nebulizing a liquid, as explained above. The aerosol nebulizer system 30 may alternatively be provided with an alternative aerosol source for dispensing the aerosol, for example into the mouthpiece of the aerosol nebulizer system. Such an alternative aerosol source may, for example, be a passive aerosol source (such as an aerosol container or the like and a mechanism to release the aerosol). The aerosol source may be a nebulizer, an atomizer, such as a humidifier, a pneumatic nebulizer, an electronic nebulizer, an ultrasonic nebulizer, an electro-hydrodynamic nebulizer, an electrostatic nebulizer, a jet nebulizer, a humidifier-nebulizer for ventilation devices, a metered-dose inhaler (MDI), a dry-powder inhaler (DPI), a spacer with MDI, a valved holding chamber with MDI or the like. Preferably the aerosol generator is a membrane nebulizer, more preferably a vibrating membrane nebulizer, e.g. an electronic vibrating membrane nebulizer.

The aerosol nebulizer system 30 is further provided with a storage unit 32 for storing a plurality of aerosol nebulizer data units or usage data units respectively related to a usage of the aerosol nebulizer system 30, wherein each of the aerosol nebulizer data units being stored in association with a corresponding identification.

That is, the aerosol nebulizer data units (also referred to as usage data units) are stored in the storage unit 32 of the aerosol nebulizer system 30, whereby the storage may be associated or be a part of the processing unit 32. The storage unit may include a RAM or other type of dynamic or static storage device that may store information and instructions for execution by the processing unit 32.

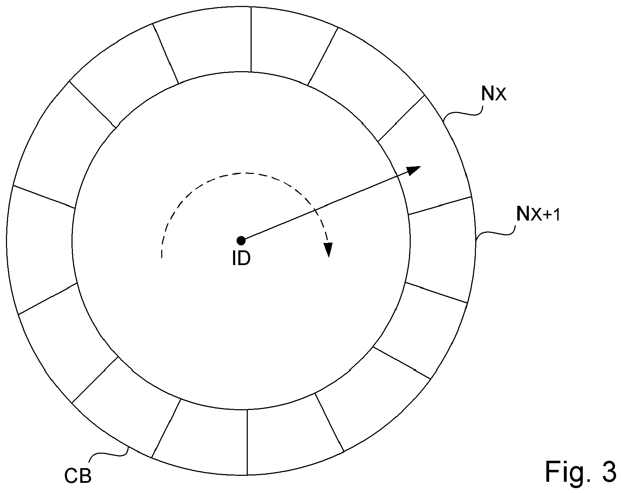

**Fig. 3** shows the example of a ring storage unit (also referred to as circular buffer, CB, in the following) as the storage unit 32, but the present invention is not restricted to using a ring storage unit. The skilled person understands that the CB stores data continuously for a certain period of time and overwrites it after a specified time has elapsed or if all storage locations are used in order to free up storage space for new data.

In the embodiment shown in **Fig. 3**, CB denotes a circular buffer providing storage locations N for storing respective aerosol nebulizer data units u(x), u(x+1) ... indicating the aerosol nebulizer usage data (as explained above) when using the aerosol nebulizer system. Here, the aerosol nebulizer data units u(x), u(x+1) ... are stored in respective storage locations Nx, Nx+1 corresponding to respective usages of the aerosol nebulizer system. As shown in **Fig. 3**, the storage location Nx, into which an aerosol nebulizer data unit corresponding to the usage has been written, may be identified by an identifier ID, so that in a subsequent usage cycle the controller of the CB stores a new aerosol nebulizer data unit in a subsequent one Nx+1 of said storage locations.

By using a circular buffer CB for storing the aerosol nebulizer data units, the memory requirements of the aerosol nebulizer are limited and impose reduced constraints on the design of the aerosol nebulizer system 30. The circular buffer CB can be embodied in various forms, for example a random access memory, preferably of the non-volatile type like a NAND flash memory. The circular buffer CB needs not to be embodied in a separate component as it is possible for those skilled in the art to implement the circular buffer CB in the processing unit 32 by means of software under the control of processing unit 32.

At the end of each of the above usage/measurement cycles, the processing unit 32 may update the identifier ID to point at the next storage location (Nx+1) so that the identifier ID may indicate the storage location of the last stored data element, which corresponds to the usage of the aerosol nebulizer system during said usage/measurement cycle. That is, an aerosol nebulizer data unit may include one or more data sets related to the usage of the aerosol nebulizer system, and may be related to different usages of the aerosol nebulizer system in the usage/measurement cycle (e.g. two or more of usage data related to inhalation treatments, sensor data, cleaning data, charging data, status data, user identification data).

The communication interface 33 will transmit aerosol nebulizer data, i.e. respective aerosol nebulizer data units u(x), u(x+1) ..., which have been newly generated/stored since the last establishment of the first wireless communication connection to the communication unit 21, 21a of the external control device 20, 20a as soon as the first wireless communication connection is again established (e.g. when switching on the control device 20, 20a, starting the APP or the like, or alternatively when establishing a direct radio connection to the server).

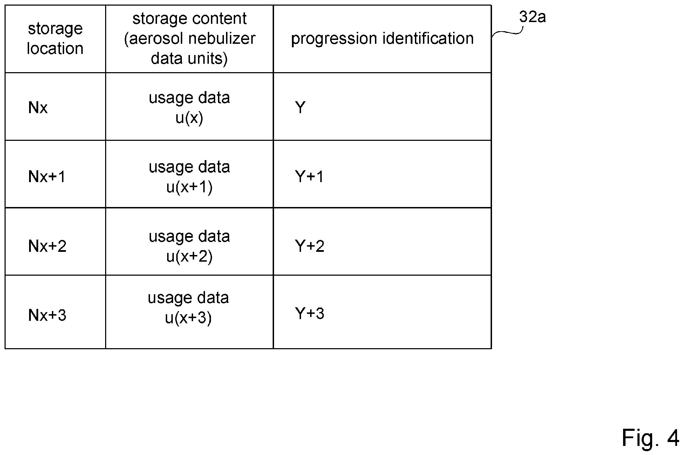

**Fig. 4** shows an example of a data structure 32a being stored in the storage unit 32 of the aerosol nebulizer system 30, for example in the CB described above. As indicated in **Fig. 4**, the data structure defines an association between storage locations Nx, Nx+1, ... (which may also be referred to as storage addresses) and respective aerosol nebulizer data units u(x), u(x+1) .... As explained, each respective aerosol nebulizer data unit includes usage data of the aerosol nebulizer system which are stored during and/or after a usage of the aerosol nebulizer system and may include one or more data of usage time, usage parameter (nebulization, cleaning, configuration update, status, user recognition, geographic position, and the like), sensor data, and the like. In addition, the data structure also defines an association of storage locations, usage data units, and respective identifications Y, Y+1, ... of the respective aerosol nebulizer data units u(x), u(x+1), .... Here, the respective identifications may be a series of integer numbers or the like, such as indicated in the following Table 1:

| storage location | aerosol nebulizer data units | Identifications |
|---|---|---|
| Nx | u(x) | 1 |
| Nx+1 | u(x+1) | 2 |
| Nx+2 | u(x+2) | 3 |
| Nx+3 | u(x+3) | 4 |
| Nx+4 | u(x+4) | 5 |

The skilled person understands that the identifications (also referred to as security codes, data integrity codes, data integrity identifications) identify a particular sequence of aerosol nebulizer data units, i.e. a specific order or consecutive numbering in which the aerosol nebulizer data units have been generated and stored. In other words, the identifications identify a consecutive sequence/order according to which the aerosol nebulizer data units are generated/stored; here u(x) first, followed by u(x+1), u(x+2), u(x+3), u(x+4) in that order. The skilled person understands that the above example of a sequence of identifications (using integer numbers) is one example, and other sequences may be used instead.

Although not shown in **Fig. 4**, the identifications may also be stored in connection with a serial number of the aerosol nebulizer system (or the aerosol generator) to provide a unique identification of the respective aerosol nebulizer data.

The aerosol nebulizer data units and corresponding identifications are transmitted when the first wireless communication connection is established. As explained above, the first wireless communication connection may not be established at all times, and, in particular, may only be established irregularly. This means that the aerosol nebulizer system may be used by a user/patient multiple times or the aerosol nebulizer system has operated, for example in a stand by operation, to clean the aerosol generator, to charge the battery, or the like, and thus a plurality of aerosol nebulizer data units may have been stored but have not been transmitted to the control device 20, 20a yet. This also means that the control unit 20, 20a does not have prior knowledge as to when aerosol nebulizer data units may be expected.

In such a situation, without providing the above identifications, the control unit 22, 22a does not have a verification mechanism as to whether all generated aerosol nebulizer data units indeed have been received. For example, if the control device merely receives the usage data units u(x), u(x+2), u(x+3), and u(x+4) of Table 1 (e.g. in a plurality of data packets) without receiving u(x+1), the control units would not have a verification mechanism that the data unit u(x+1) is missing. It is noted that such an information may also not be derivable in case an aerosol nebulizer usage time is stored in the aerosol nebulizer data units, in particular because the user may also use aerosol generator at irregular times.

In view of these circumstances, the present inventors have implemented a new data integrity mechanism by which the control unit 22, 22a of the control device 20, 20a (mobile device or alternatively the server, if the aerosol nebulizer system directly communicates with the server via a radio connection) is configured to evaluate a progression of identifications received via the first data transmission and received in association with aerosol nebulizer data units respectively related to a usage of the aerosol nebulizer system 30.

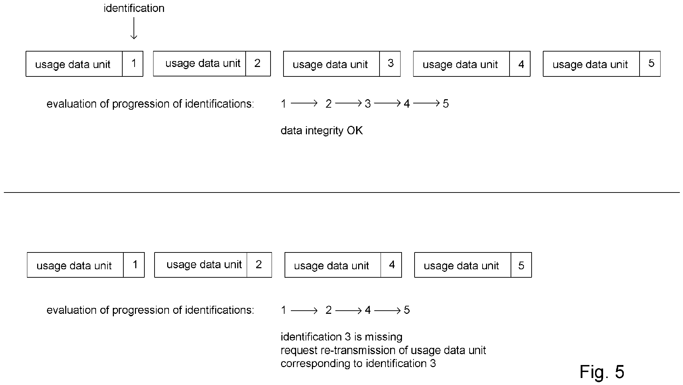

Here, a received progression of identifications refers to the sequence of identifications received in conjunction or in association with respective aerosol nebulizer data units. Applying the example of Table 1 described above, such a progression or sequence of received identifications may be, according to a first example: 1, 2, 3, 4, 5, and may be, according to a second example: 1, 2, 4, 5, as shown in **Fig. 5** which shows a conceptional overview of the data integrity mechanism for a plurality of usage data units respectively received (e.g. via a plurality of data packets) in conjunction with corresponding data integrity identifications.

The control unit 22, 22a (in the mobile device or the server, e.g. backend server system, 40 (as further described above)) is configured to evaluate this progression. In the first example in **Fig. 5**, the control unit 22, 22a recognizes that a complete progression (without missing identification) is received so that the usage data transmission over the first wireless communication connection is complete. On the other hand, in the second example in **Fig. 5**, the control unit 22, 22a recognizes that an incomplete progression (missing identification 3) has been received so that the usage data transmission over the first wireless communication connection is not complete. Based on this new data integrity mechanism, the control unit 22, 22a can determine whether all aerosol nebulizer data units have been successfully transmitted to the control device 20, 20a and data integrity with regard to an entire sequence/progression of usage data has been achieved. In the second example of **Fig. 5**, the control unit 22, 22a may thus determine that there is a data integrity failure, in particular because the evaluation of the progression of identifications indicates that there is a missing identification in the identification sequence.

The skilled person recognizes that such a (second) data integrity mechanism improves over the error correction (checksum or the like) provision with regard to a single data unit, referred to as first data integrity mechanism above. Such a first data integrity mechanism can only be applied if the control unit 22, 22a has actually received the single data unit (for example with some transmission error), because the checksum has to be evaluated. The skilled person understands, however, that this mechanism does not work if the control unit 22, 22a has no prior knowledge as to when a particular usage data unit should be received, as it may be the case in the present environment of irregular aerosol nebulizer usage and irregular establishment of the first wireless communication connection.

According to another embodiment, the control unit 22, 22a may, as shown in **Fig. 5**, be further configured to request a re-transmission of a specific aerosol nebulizer data unit which has been determined to be missing in the progression of identifications. That is, in the above example, the control unit 22, 22a evaluates the received progression of identifications 1, 2, 4, 5 and thus determines that the aerosol nebulizer data unit associated with the identification "3" is missing. As such, the control unit 22, 22a may request a re-transmission of the missing aerosol nebulizer usage data via the established first wireless communication connection. The control unit 22, 22a may repeat this re-transmission request several times, in particular also after the first wireless communication connection has been disconnected and re-established again. As such, the control unit 22, 22a is provided with a mechanism to receive a complete set of aerosol nebulizer data units.

The aerosol nebulizer communication interface 33 which is configured to establish the first wireless communication connection, as explained above, and may thus receive the re-transmission request of one or more specific aerosol nebulizer data units which has/have been determined to be missing in the progression of identifications. Using the example of the missing identification "3" explained above, when receiving the request to re-transmit the aerosol nebulizer data unit associated with the missing identification "3", the processing unit 32 may operate to access the storage unit (e.g. CB in **Fig. 3**) and retrieve the specific aerosol nebulizer data unit associated with the missing identification "3". Subsequently, the retrieved specific aerosol nebulizer data unit is transmitted in combination with the missing identification by the aerosol nebulizer communication interface 33 to the control device 20, 20a.

The skilled person understands that a complete set of aerosol nebulizer data units guarantees that all nebulizer usage data are taken into account when evaluating adherence to a prescribed therapy plan and/or when determining a specific change to the therapy plan and/or device status and/or user based on the aerosol nebulizer usage data (for example, to reduce or increase the number of inhalation treatments depending on an evaluation of sensor data). Advantageously, a change to the therapy plan is only made when a complete set of aerosol nebulizer data units is available, i.e. not when it is determined that one or more aerosol nebulizer data units are missing. In other words, successful reception of missing (re-transmitted) aerosol nebulizer data units will be awaited before making a change to the therapy plan. This guarantees that a change of the therapy plan is based on a complete set of aerosol nebulizer data units.

According to a further embodiment, even if a determination is made that one or more identifications are missing in the progression of the identifications, the received usage data units are used for adherence evaluation without awaiting re-transmission of the missing usage data units. In other words, even if usage data units are determined to be missing, the control device 20, 20a (mobile device or server) may already process the already received usage data units (i.e. usage data units 1, 2, 4, and 5 in the example of Fig. 5) to evaluate therapy adherence based on this limited set of information (without waiting for the reception of a missing usage data unit). This is of particular advantage as a successful re-transmission of a missing usage data unit may be delayed (e.g. because the aerosol nebulizer may already be switched off or the like) and even a limited adherence evaluation may already indicate that or to what extent or to what percentage the user is following the therapy protocol (while still awaiting usage data that are in the process of being re-transmitted).

According to a further embodiment, the control unit 22a may also be configured to forward the identifications and corresponding or associated aerosol nebulizer data units (in the following also referred to as usage data units) to the external device 40, i.e. a mobile device or server. Such forwarding may be performed without a permanent storage (in a cache or the like) of the identifications and corresponding/associated aerosol nebulizer data units at the control device or with a permanent storage or without waiting for the re-transmission of missing aerosol nebulizer data units. This forwarding is performed via the second wireless communication connection explained above and provides the usage data units to the external device 40 for subsequent and rapid evaluation of the adherence to the therapy protocol. This forwarding may also include an information as to missing aerosol nebulizer data units, so that the external device 40 is informed about the fact that missing aerosol nebulizer data units in the progression will be provided.

Here, the control unit 22a may alternatively await re-transmission of one or more missing aerosol nebulizer data units and forward the complete set of missing aerosol nebulizer data units according to the progression of identifications.

Forwarding the identifications and corresponding or associated aerosol nebulizer data units to the external device 40 is also implemented in view of the fact that the aerosol nebulizer system may be controlled via one or more control devices each operating under control of the same APP. For example, a user may establish a first wireless communication connection using the smartphone in the morning while establishing a first wireless communication connection using a tablet in the evening. In such a case, transmission of aerosol nebulizer data units may occur to the smartphone and the tablet, each having a risk of data loss and only partial data storage.

According to another embodiment, the control unit 22, 22a or the external device 40 may also be configured to trigger an issuance of a notification to the user of the control device 20, 20a or the external device 40 to switch on the aerosol nebulizer system 30 when a specific time period is expired. Such a notification may also or alternatively be provided to an external terminal device being related to an external care giver, a smart home environment, a monitoring station of a nursing home or the like, a family member, parents, friends, an insurance company or the like. Here, the specific time period may be a predetermined time period, for example a time period during which the user/patient is expected to perform a specific number of inhalations according to the therapy protocol. The issuance of such a notification reminds the user to switch on the aerosol nebulizer system 30 in order to establish the first wireless communication connection so that the stored aerosol nebulizer data units (which are not yet transmitted to the control device 20, 20a) can be transmitted. Providing the notification to a third terminal device, as explained above, may support such a reminder mechanism. As a result, the control device 20, 20a is provided with up-to-date aerosol nebulizer data units and can timely monitor the adherence to the prescribed therapy protocol and/or the status of the device and/or the user of the aerosol nebulizer system.

Advantageously, the sharing of notifications or specific reports (as described below) enables to include other people of choice associated with the user/patient as support in their therapy management and monitoring.

Preferably, settings for the reminder may be personalized, e.g. personalized reminder message (based on user character, daily mood and external factors) and/or medication specific. Such reminders may also only be provided with regard to non-routine medication, in order to minimize the number of reminder alerts for the user. According to another embodiment, the control unit 22, 22a or the external third device 40 may be further configured to allow a user to select a specific report generating functionality. Such specific report generating functionalities may be related to respective different recipients, such as a physician, parents, the insurance, friends, or the like. This mechanism allows to provide different recipients with corresponding specific reports for which a different set of aerosol nebulizer data units may be required. In this context the control unit 22a (e.g. mobile device) may be configured to request a transmission of report-related inhalation data units stored at the third device 40 (e.g. server). For example, if the user wishes to provide a report (i.e. a therapy adherence report) to the physician, the control unit 22a may request all relevant usage data, in particular including also sensor data, from the third device 40 to be included in the report. On the other hand, if the user wishes to provide a report to a family member or friend, who supports the therapy of the user, a detailed report may not be required and thus only a reduced set of aerosol nebulizer data units need to be transmitted from the third device 40.

The external device 40 (either a mobile device or a server, as explained above), illustrated in **Fig. 2**, has a communication interface 41 which is configured to establish the second wireless communication connection, as explained above, with one or more control devices 20a, as explained above, and to perform second data transmission with the one or more control devices 20a. Here, the one or more control devices 20a may be related to a plurality of portable or mobile devices such as a smartphone, a tablet, a wearable device, a voice-controlled device and the like on which the same APP is implemented.

Further, a control unit 42 of the external device 40 in **Fig. 2** may be configured to evaluate a progression of identifications of aerosol nebulizer data units respectively related to a usage of the aerosol nebulizer system 30. The evaluation of the progression of identifications may be performed as explained above for the control device 20, 20a and is performed to guarantee that a complete set of aerosol nebulizer data units is used for evaluating the adherence to the therapy protocol. Such an additional evaluation of the progression of identifications is performed as also the second wireless communication connection may be prone to data loss and due to the fact that the aerosol nebulizer data units may be transmitted from different control devices 20, 20a. The evaluation with the control unit 42 could be an additional or an alternative method to proof data integrity.

According to another embodiment, the control unit 42 may be further configured to request a re-transmission of a specific aerosol nebulizer data unit which has been determined at the external device 40 to be missing in the progression of identifications. Such a re-transmission request may be transmitted to (any of) the control device (s) 20a. If the missing aerosol nebulizer data unit(s) is (are) available at the control device 20a, then the control device transmits the missing aerosol nebulizer data unit(s) to the external device 40. On the other hand, if the missing aerosol nebulizer data unit(s) is (are) not available at the control device 20a, because it has been discarded from temporal memory or because the missing aerosol nebulizer data unit(s) have initially been transmitted to a different control device 20a, then the control device 20a requests a re-transmission from the aerosol nebulizer system 30, as explained above.

According to another embodiment, the control unit 42 may be further configured to trigger an issuance of a request to transmit further aerosol nebulizer data unit(s) (e.g. inhalation data units, sensor data units, or the like) when a specified time period is expired. This request may be issued to the control device(s) 20a as described above or another mobile device, for example by a PUSH notification, by e-mail, using an instant messaging service, or the like. Such a specified time period may be a time period at which a user/patient is expected to have been performed a specified number of further inhalations according to the therapy plan. Such a reminder function may thus be used to cause the user/patient to switch on the control device 20a so that the newly generated aerosol nebulizer data units are transmitted. This further supports adherence to the therapy plan and timely evaluation of the aerosol nebulizer data. Such a timely evaluation of the aerosol nebulizer data may be specifically advantageous in cases where the medical condition of a user/patient becomes worse and an immediate adaptation of the therapy plan becomes necessary.

Advantageously, the above mechanism also encourages the user/patient to engage with the control device 20, 20a (e.g. the APP) to regularly transfer aerosol nebulizer data from the aerosol nebulizer system to the server. This mechanism may be further enhanced using gamification or the like, in particular for children or elderly people which may be of need of providing enhanced reminder functionality.

According to another embodiment, the control unit of the control device 20, 20a (mobile device or server) or the control unit of the external third device 40 (e.g. the server) may be further configured to determine an adherence-related patient type based on one or more machine-learning algorithms. Here, an adherence-related patient type may be defined as respective patient types for which an adherence behaviour is similar and/or for which respective factors influencing the adherence are similar. For example, specific ranges of therapy adherence may be defined, e.g. adherent below a 50% range, between a 50% and 75% range, or above a 75% range or near a 100%, or patient types reacting to reminders within several different time ranges. Other examples of factors influencing the way how to motivate patients to be more adherent are number of necessary treatments, progression of disease, family status and age.

Depending on such a determination, the time, way (e.g. number of receivers), periodicity, frequency with regard to the issuance of reminders may be selectively set for the specific adherence-related patient type.

Advantageously, the specific adherence-related patient type may be determined with regard to one or more of a course of disease, age, gender, lung function, weight. The skilled person understands that data of additionally connected devices, activity profiles and the like, may also be used as an input to the machine-learning algorithms.

According to another embodiment, the control unit of the control device 20, 20a (mobile device or server) or the control unit of the external third device 40 (e.g. the server) may further be configured to determine an appropriate adherence stimulus, in particular with regard to the determined adherence-related patient type, based on the one or more machine-learning algorithms. Here, the adherence stimulus may be a specific way to support or advance the adherence to the therapy plan, and may include a specific motivation for preventive action (physical action, sport activities, eating or drinking habits, sleeping patterns or the like). Depending on the current health condition, a trend or change in a health status, as determined by the control device 20, 20a (mobile device or server) or third device 40 based on the aerosol nebulizer data units, a different adherence stimulus may be determined and provided to the control device 20, 20a. This may also be connected with the report generating functionality as explained above, so that a specific adherence stimulus is also included in such reports which may preferably be provided to another individual (a buddy system) to support the user/patient in following the therapy plan.

According to another embodiment, the control unit of the control device 20, 20a (mobile device or server) or the control unit of the external third device 40 (e.g. the server) may be further configured to trigger an escalation chain if the user/patient does not or continues to not transmit aerosol nebulizer data units and/or if the user/patient does not or continues to not adhere to a prescribed therapy protocol. Alternatively, the escalation chain may also be triggered if the evaluation of the aerosol nebulizer data units indicates a change in the patient status or a change in the nebulizer status.

Such an escalation chain is a technical mechanism by which the amount, frequency, and/or number of recipients is increased over time, and thus indicates an increasing urgency to use the aerosol nebulizer system. Such an escalation chain may be advantageously be determined based on different thresholds with regard to actual adherence, e.g. adherent within a 25% range, within a 50% range, within a 80% range.

A specific example of such an escalation scheme may be as follows:
1. Push message to user/patient
2. e-mail to the user/patient
3. push message and/or e-mail to the user/patient, indicating that a third person (e.g. preferred support contact) will be informed
4. e-mail to the third person

The skilled person understands that this is just an example of an escalation scheme and other escalation schemes are possible.

A fluid or liquid to be nebulised or aerosolised by the aerosol generator or as a basis the aerosol may be a fluid or liquid for the generation of a pharmaceutical aerosol for the delivery of an active compound.

An active compound is a natural, biotechnology-derived or synthetic compound or mixture of compounds useful for the diagnosis, prevention, management or treatment of a disease, condition or symptom of a mammal, in particular a human. Other terms which may be used as synonyms of active compounds include, for example, active ingredient, active pharmaceutical ingredient, drug substance, diagnostic material, drug, medicament and the like. The fluid could be of a liquid, solution, suspension, colloidal mixture or liposomal formulation form and can be prepared, mixed or opened before or during the application.

The active compound comprised in the fluid to be nebulised or aerosolised by the aerosol generator may be a drug substance or a medicament which is useful for the prevention, management, diagnosis or treatment of any disease, symptom or condition affecting the body, skin, body cavities, the abdomen, the eyes, the ear, the intestine, the stomach, the nose, the nasal cavities, the sinuses, the osteomeatal complex, the mouth, the trachea, the lungs, upper lungs, lower lungs, central lungs, the bronchia, the bronchioles, the alveoli and/or the respiratory tract. In particular an aerosol comprising an active compound, which is useful for the prevention, management, diagnosis or treatment of any pulmonary or respiratory disease, symptom or condition. The active compound comprised in the fluid to be nebulised or aerosolised by the aerosol generator may be used especially for clinical trials or regulatory approvals.

Among the active compounds which may be useful for serving one of the purposes named previously and that may be used together with the present invention, are, for example, substances selected from the group consisting of anti-inflammatory compounds, anti-infective agents, antiseptics, prostaglandins, endothelin receptor agonists, phosphodiesterase inhibitors, beta-2-sympathicomimetics, decongestants, vasoconstrictors, anticholinergics, immunomodulators, immunoglobulins, mucolytics, anti-allergic drugs, antihistaminics, mast-cell stabilising agents, tumor growth inhibitory agents, wound healing agents, local anaesthetics, antioxidants, oligonucleotides, peptides, proteins, vaccines, vitamins, plant extracts, cholinesterase inhibitors, vasoactive intestinal peptide, serotonin receptor antagonists, and heparins, glucocorticoids, anti-allergic drugs, antioxidants, vitamins, leucotriene antagonists, anti-infective agents, antibiotics, antifungals, antivirals, mucolytics, decongestants, antiseptics, cytostatics, immunomodulators, vaccines, wound healing agents, local anaesthetics, oligonucleotides, xanthin derived agents, peptides, proteins and plant extracts. Such compound may be used in the form of a suspension, a solution, a colloidal formulation (i.e., liposomal), etc.

Examples of potentially useful anti-inflammatory compounds are glucocorticoids and non-steroidal anti-inflammatory agents such as arformoterole, betamethasone, beclomethasone, budesonide, ciclesonide, dexamethasone, desoxymethasone, fluoconolone acetonide, fluocinonide, flunisolide, fluticasone (propionate), formoterole, fumarate, icomethasone, rofleponide, tiotropium, triamcinolone acetonide, fluocortin butyl, hydrocortisone, hydroxycortisone-17-butyrate, prednicarbate, 6-methylprednisolone aceponate, mometasone furoate, pirfenidone, dehydroepiandrosterone-sulfate (DHEAS), tartrate, umeclidinium, vilanterol, elastane, prostaglandin, leukotriene, bradykinin antagonists, non-steroidal anti-inflammatory drugs (NSAIDs), such as ibuprofen and acetylsalicylic acid (ASA), including any pharmaceutically acceptable salts, esters, isomers, stereoisomers, diastereomers, epimers, solvates or other hydrates, prodrugs, derivatives, or any other chemical or physical forms of active compounds comprising the respective active moieties and combination thereof, like for example LABA and LAMA combination like aclidinium and formoterol.

Examples of anti-infective agents, whose class or therapeutic category is herein understood as comprising compounds which are effective against bacterial, fungal, and viral infections, i.e. encompassing the classes of antimicrobials such as for example bacteriophages (for example the treatment of pulmonary infections in cystic fibrosis patients) or antimicrobial petides (for example for the treatment of MDR G negative Pneumonia), antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam);
- cephalosporins, including cefazolins (cefazolin, cefazedone); cefuroximes (cefuroxim, cefamandole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;
- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals, antimycotics, fungicide or fungistatic, such as for example flucytosin, griseofulvin, tolnaftal, naftifin, terbinafin, amorolfin, ciclopiroxolamin, echinocandins, such as micafungin, caspofungin, anidulafungin, amphotericin B or variconazole;
- nitrofurans, including nitrofurantoin and nitrofuranzone;
- polyenes, including amphotericin B, natamycin, nystatin, flucytosine;
- other antibiotics, including tithromycin, lincomycin, clindamycin, oxazolindiones (linzezolids), ranbezolid, streptogramine A+B, pristinamycin A+B, Virginiamycin A+B, dalfopristin /quinupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, formycin, pentamidine, and Fab-I-Inhibitors;

Usful drugs may be afabicine and/or derivates thereof;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors, siRNA based drugs;
- antiseptics, including acridine derivatives, iodine-povidone, benzoates, rivanol, chlorhexidine, quarternary ammonium compounds, cetrimides, biphenylol, clorofene, and octenidine;
- plant extracts or active ingredients of plants, such as plant extracts from chamomile, hamamelis, echinacea, calendula, thymian, papain, pelargonium, pine trees, essential oils, myrtol, pinen, limonen, cineole, thymol, mentol, camphor, tannin, alpha-hederin, bisabolol, lycopodin, vitapherole; useful drugs may be plant extracts of cannabinoids, active ingredients of cannabinoids, such as for example terahydrocannabinol, and/or derivates of active inredients of cannabinoids;
- wound healing compounds, including pirfenidone, dexpantenol, allantoin, vitamins, hyaluronic acid, alpha-antitrypsin, anorganic and organic zinc salts/compounds, salts of bismuth and selen;
- Useful drugs may be syntetic analogues of prostacyclines and/or derivates thereof;

Useful drugs may be gluthathione and/or derivates thereof;
- antifibrotic compounds, for example, pirfenidone;
- interferones (alpha, beta, gamma), tumor necrosis factors, cytokines, interleukines;
- immunomodulators, including immunosuppressive compounds, antibody (Ab), cytostatics and metastasis inhibitors;
- immunosuppressive compounds, including glucocorticoids, cytostatics, drugs acting on immunophilins or on other drugs, such as interferons, opioids, TNF binding proteins or mycophenolate;
- immunosuppressive compounds, including immunomodulating agents, or immunosuppressive agents or antirejection medications are compounds that may for example inhibit or prevent activity of the immune system, for example to prevent graft rejection;
- immunosuppressive compounds, including drugs acting on immunophilins, such as ciclosporin, tacrolimus, sirolimus, everolimus, mycophenolic acid, also called mycophenolate or mycophenolate-mofetil, methotrexat, or azathioprine;
- antibody (Ab), including polyclonal antibodies or monoclonal antibodies, such as for for example immunoglobulin (Ig), immunoglobulin G (IgG - for example Privigen - IgPro10), immunoglobulin A (IgA), or immunoglobulin M (IgM); as well as fragments of antibodies, also known as Fab (fragment, antigen-binding) region, complementarity determining regions (CDRs), Fc (Fragment, crystallizable) region, or Fc receptors; or nanobodies for example for the treatment of RSV;
- cytostatics and metastasis inhibitors, including chemotherapeutic agents or anti-cancer drugs, such as alkylating agents, antimetabolite, antimicrotubuli agents, anthracyclines, cisplatin, cyclophosphamid, cytoskeletal disruptors (taxanes), epothilones, histone deacetylase inhibitors, ifosfamid, inhibitors of topoisomerase I + II, intercalating agents, kinase inhibitors, mitomycin, nucleotide analogs and precursor analogs, peptide antibiotics, nanododies, platinum-based agents, retinoids, and vinca alkaloids and derivatives;
- alkylating agents, such as nimustine, melphanlane, carmustine, lomustine, cyclophosphosphamide, mechlorethamine, dacarbazine, nitrosoureas, temozolomide (oral dacarbazine), ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- taxane, such as paclitaxel, abraxane, taxotere or docetaxel;
- topoisomerase I inhibitors, such as topotecan or irinotecan;
- topoisomerase II inhibitors, such as doxorubicin or etoposid;
- intercelating agents, such as anthracyclines, like doxorubicin;
- platinum-based agents, such as cisplatin, carboplatin, oxaliplatin, or satraplatin;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of transition group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, docetaxel, gefitinib, vandetanib, erlotinib, poly-ADP-ribose-polymerase (PRAP) enzyme inhibitors, banoxantrone, gemcitabine, pemetrexed, bevacizumab, ranibizumab.

Examples of potentially useful mucolytics are DNase, P2Y2-agonists (denufosol), drugs affecting chloride and sodium permeation, such as sodium chloride (NaCl, e.g., 0.9%, 3%, 6%, 7% solutions), ectoine (1,4,5,6-tetrahydro-2-methyl-4-pyrimidinecarboxylic acid), N-(3,5-Diamino-6-chloropyrazine-2-carbony)-N'-{4-[4-(2,3-dihydroxypropoxy)-phenyl]butyl}guanidine methanesulfonate (PARION 552-02), heparinoids, guaifenesin, acetylcysteine, carbocysteine, ambroxol, bromhexine, tyloxapol, lecithins, myrtol, surfactant, synthetic surfactant and recombinant surfactant proteins.

Example for a pulmonary surfactant (derived from: surface active agent), which support pulmonary development, may be, an exogenous pulmonary surfactant, or belong to the class of "modified natural" pulmonary surfactants, which are lipid extracts of minced mammalian lung or lung lavage. These preparations have variable amounts of SP-B and SP-C proteins and, depending on the method of extraction, may contain non-pulmonary surfactant lipids, proteins or other components. Some of the modified natural pulmonary surfactants present on the market, like Survanta™, are spiked with synthetic components such as tripalmitin, dipalmitoylphosphatidylcholine and palmitic acid.

Example of current modified natural pulmonary surfactants include, but are not limited to, bovine lipid pulmonary surfactant (BLES™, BLES Biochemicals, Inc. London, Ont), calfactant (Infasurf™, Forest Pharmaceuticals, St. Louis, Mo.), bovactant (Alveofact™, Thomae, Germany), bovine pulmonary surfactant (Pulmonary surfactant TA™, Tokyo Tanabe, Japan), and beractant (Survanta™, Abbott Laboratories, Inc., Abbott Park, Ill.).

Example of pulmonary surfactant, which may belong to the class of "artificial" pulmonary surfactants, are simply mixtures of synthetic compounds, primarily phospholipids and other lipids that are formulated to mimic the lipid composition and behaviour of natural pulmonary surfactant and are devoid of pulmonary surfactant proteins, are artificial surfactants include, but are not limited to, pumactant (Alec™, Britannia Pharmaceuticals, UK), and colfosceril palmitate (Exosurf™, GlaxoSmithKline, plc, Middlesex).

Example of pulmonary surfactant, which may belong to the class of "reconstituted" pulmonary surfactants, are artificial pulmonary surfactants to which have been added pulmonary surfactant proteins/peptides isolated from animals or proteins/peptides manufactured through recombinant technology such as those described in WO 95/32992, or synthetic pulmonary surfactant protein analogues such as those described in WO 89/06657, WO 92/22315 and WO 00/47623, are reconstituted surfactants include, but are not limited to, poractant alfa (Curosurf™ Chiesi Farmaceutici S.p.A.) and lucinactant (Surfaxin™, Windtree Therapeutics, Inc., Warrington, Pa.) and the product having the composition disclosed in WO 2010/139442.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine.

Examples of potentially useful local anaesthetic agents include benzocaine, tetracaine, procaine, lidocaine and bupivacaine.

Examples of potentially useful antiallergic agents or anti-asthma compounds include the afore-mentioned glucocorticoids, cromolyn sodium, nedocromil, cetrizin, loratidin, montelukast, roflumilast, ziluton, omalizumab, heparinoids and other antihistamins, including azelastine, cetirizin, desloratadin, ebastin, fexofenadin, levocetirizin, loratadin.

Examples of potentially useful anticholinergic agents include ipratropium bromide, tiotropium bromide, oxitropium bromide, glycopyrrolate.

Examples of potentially useful beta-2-sympathicomimetic agents include salbutamol, fenoterol, formoterol, indacaterol, isoproterenol, metaproterenol, salmeterol, terbutaline, clenbuterol, isoetarine, pirbuterol, procaterol, ritodrine and long-acting beta-agonists (LABAs) such as Albuterol sulphate, formoterol fumarate, salmeterol xinafoate, arformoterol tartrate, and olodaterol.

Examples of potentially useful muscarinic antagonists are muscarine and nicotine, such as ipratropium bromide and acetylcholine as well as long-acting muscarinic antagonists (LAMA) such as aclidinium (bromide), glycopyrronium (bromide), ipratropium, tiotropium (bromide), and umeclidinium (bromide).

Examples of xanthine derived agents include theophylline, theobromine, caffeine.

Example of PDE5-Inhibitor include sildenafil.

Example of antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides dependent on their composition useful for the treatment of many diseases and various compounds are currently clinically evaluated, such as ALN-RSV01 to treat the respiratory syncytical virus by, AVE-7279 to treat asthma and allergies, TPI-ASM8 to treat allergic asthma, 1018-ISS to treat cancer. Examples of potentially useful peptides and proteins include antibodies against toxins produced by microorganisms, antimicrobial peptides (for example for the treatment of MDR G negative Pneumonia), such as cecropins, defensins, thionins, and cathelicidins.

Example of radioactive agents for diagnoses or clinical trials, such as technetium 99m [Tc99, Technegas, Technetium (99mTc), Technetium-99 (99Tc)], krypton (81mKr) inhalation gas, and Xenon-133 [Xenon Xe-133]. A number of isotopes, such as iodine-131 (1311), phosphorous-32 (32P), strontium-90 (90Sr), and yttrium-90 (90Y), may be used. Especially for a pulmonary ventilation and blood perfusion (V/Q) diagnose scan or scintigraphic pulmonary deposition studies the isotopes, krypton (81mKr) inhalation gas or technetium 99m (99mTc), may be used.

Example of potentially useful opioids, such as endogenous opioids, opium alkaloids and derivatives, synthetic opioids, allosteric modulators, and opioid antagonists.

Useful drugs may be biosimilars such as for example Dornase-Alpha for cystic fibrosis patients;

The nebulizer may be used with fluids or liquids of the groups of viral gene therapy agents or non-viral gene therapy agents. The transferred nucleotide constructs may be single or double stranded DNA, RNA, or siRNA. In one study the gene therapeutic agent carries especially the CF gene to substitute and cure the cystic fibrosis deficiency. For the transfer to the patient the substitute is integrated in a viral vector and masked in liposomes. The, from UK CF Gene Therapy Consortium (GTC) called, inhalative gene therapeutic agent "pGM169/GL67A" is under clinical evaluation.

The above respective units of the control device, the aerosol nebulizer system, and the server may thus be implemented by a respective processing unit (CPU) that include one or a plurality of processors, a microprocessor or other processing logic that interprets and executes instructions as defined by the computer program and stored in a main memory. The main memory (storage unit) may include a RAM or other type of dynamic storage device that may store information and instructions for execution by the respective modules/units. For example, the evaluation unit and/or the sensor data control unit discussed above may be realized by the processing unit. The ROM may include a ROM device or another type of static storage device that may store static information and instructions for use by the processing unit.

The control device 20, 20a for example, may perform these operations in response to the processing unit executing software instructions contained in a computer-readable medium, such as the main memory, ROM and/or storage device. A computer-readable medium may be defined as a physical or a logical memory device. For example, a logical memory device may include memories within a single physical memory device or distributed across multiple physical memory devices. Each of the main memory, ROM and storage device may include computer-readable media with instructions as program code. The software instructions may be read into the main memory for another computer-readable medium, such as a storage device or from another device via the communication interface.

The software instructions of a computer program (e.g. mobile application, APP) contained in the main memory, stored in a non-transitory computer-readable storage medium, or provided via a download (through a corresponding signal) may cause the control or processing unit(s) including a data processor, when executed on the processing unit, to cause the data processor to perform operations or processes described herein. Alternatively, hardwired circuitry may be used in place or in combination with the software instructions to implement processes and/or operations described herein. Thus, implementations described herein are not limited to any specific combination of hardware and software.

Further, the respective units of the control device may be implemented in hardware, software, Field Programmable Gate Arrays (FPGAs), application-specific integrated circuits (ASICs), firmware or the like.

It will be apparent to those skilled in the art that various modifications and variations can be made in the entities and methods of this invention as well as in the construction of this invention without departing from the scope of the invention.

The invention has been described in relation to particular embodiments and examples which are intended in all aspects to be illustrative rather than restrictive. Those skilled in the art will appreciate that many different combinations of hardware, software and/or firmware will be suitable for practicing the present invention.

## Claims

1. Control device (20, 20a) for controlling an operation of an aerosol nebulizer system (30), said aerosol nebulizer system (30) comprising an aerosol generator (31) for nebulizing a liquid or an aerosol source for dispensing aerosol,
said control device (20, 20a) comprising:
a communication unit (21, 21a), configured to establish a first wireless communication connection and to perform first data transmission with the aerosol nebulizer system (30),
a control unit (22, 22a), configured to evaluate a progression of identifications received via the first data transmission and received in association with aerosol nebulizer data units respectively related to a usage of the aerosol nebulizer system (30).

2. Control device (20a) according to claim 1, wherein the communication unit (21a) is further configured to establish a second wireless communication connection and to perform second data transmission with an external device (40).

3. Control device (20a) according to claim 2, wherein the control unit (22a) is further configured to forward the received identifications and aerosol nebulizer data units to the external device (40).

4. Control device (20, 20a) according to any one of claims 1 - 3, wherein the control unit (22, 22a) is further configured to request a re-transmission of a specific aerosol nebulizer data unit which has been determined to be missing in the progression of identifications.

5. Control device (20, 20a) according to any of claims 1 - 4, wherein the control unit (22, 22a) is further configured to use the received usage data units for adherence evaluation without awaiting re-transmission of a missing usage data unit.

6. Control device (20, 20a) according to any of claims 1 - 5, wherein the control unit (22, 22a) is further configured to trigger an issuance of a notification to the user of the control device (20, 20a) and/or to an external terminal device to switch on the aerosol nebulizer system (30) when a specific time period is expired.

7. Control device (20a) according to any of claims 2 - 6, wherein the control unit (22a) is further configured to allow a user to select a specific report generating functionality and wherein the control unit (22) is further configured to request a transmission of report-related aerosol nebulizer data units stored at the external device (40).

8. Control device (20, 20a) according to any one of claims 1 - 7, wherein the control unit (22, 22a) is further configured to provide a plurality of specific report generating functionalities respectively related to a specific recipient.

9. Control device (20, 20a) according to any of claims 1 - 8, wherein the control unit (22, 22a) is further configured to determine an adherence-related patient type based on one or more machine-learning algorithms.

10. Control device (20, 20a) according to any of claims 1 - 9, wherein the control unit (22, 22a) is further configured to determine an appropriate adherence stimulus, in particular with regard to the determined adherence-related patient type, based on the one or more machine-learning algorithms.

11. Control device (20, 20a) according to any of claims 1 - 10, wherein the control unit (22, 22a) is further configured to trigger an escalation chain if the user/patient does not transmit aerosol nebulizer data units and/or if the user/patient does not adhere to a therapy protocol or if an evaluation of the aerosol nebulizer data units indicates a change in the patient status or a change in the nebulizer status.

12. Control device (20, 20a) according to any one of claims 1 - 11, wherein the control device (20, 20a) is a mobile device or a server.

13. Aerosol nebulizer system (30), comprising:
an aerosol generator (31) for nebulizing a liquid or an aerosol source for dispensing aerosol;
a storage unit (32) for storing a plurality of aerosol nebulizer data units respectively related to a usage of the aerosol nebulizer system (30), each of the aerosol nebulizer data units being stored in association with a corresponding identification so that a progression of identifications is stored in association with the plurality of aerosol nebulizer data units.

14. The aerosol nebulizer system (30) according to claim 13, further comprising:
an aerosol nebulizer communication interface (33) configured to establish a wireless communication connection with a control device (20, 20a) and to receive a request for re-transmission of a specific aerosol nebulizer data unit which has been determined to be missing in the progression of identifications transmitted to the control device (20, 20a).

15. The aerosol nebulizer system (30) according to claim 14, wherein
the aerosol nebulizer communication interface (33) is further configured to re-transmit the specific aerosol nebulizer data unit.

16. A server (40), comprising
a server communication interface (41) configured to establish a wireless communication connection with one or more control devices (20a) according to claims 1 - 11 and to perform data transmission with the one or more control devices (20a);
a server control unit (42) configured to evaluate a progression of identifications received via the data transmission and received in association with aerosol nebulizer data units respectively related to a usage of the nebulizer system (30).

17. The server (40) according to claim 16, wherein the server control unit (42) is further configured to request a re-transmission of a specific aerosol nebulizer data unit which has been determined to be missing in the progression of identifications.

18. The server (40) according to one of claims 16 - 17, wherein the server control unit (42) is further configured to trigger an issuance of a request to transmit further aerosol nebulizer data units when a predetermined time period is expired.

19. The server (40) according to any of claims 16 - 18, wherein the server control unit (42) is further configured to determine an adherence-related patient type based on one or more machine-learning algorithms.

20. The server (40) according to any of claims 16 - 19, wherein the server control unit (42) is further configured to determine an appropriate adherence stimulus, in particular with regard to the determined adherence-related patient type, based on the one or more machine-learning algorithms.

21. The server (40) according to any of claims 16 - 20, wherein the server control unit (42) is further configured to trigger an escalation chain if the user/patient does not transmit aerosol nebulizer data units and/or if the user/patient does not adhere to a therapy protocol or if an evaluation of the aerosol nebulizer data units indicates a change in the patient status or a change in the nebulizer status.

22. A computer program comprising instructions to cause the control device (20) of any of claims 1 - 12 to execute the steps of:
establishing a first wireless communication connection between control device (20, 20a) and nebulizer system (30) and performing first data transmission with an aerosol nebulizer system (30)
evaluating a progression of identifications received via the first data transmission and received in association with aerosol nebulizer data units respectively related to a usage of the aerosol nebulizer system (30).

23. A non-transitory computer-readable storage medium comprising the computer program of claim 22.

24. A signal carrying a computer program of claim 22.
